# EUROPÄISCHE PATENTANMELDUNG

(11) **EP 1 586 582 A1**
(43) Veröffentlichungstag der Anmeldung: **19.10.2005**
(21) Anmeldenummer: 05009073.7
(22) Anmeldetag: 03.04.2000
(51) Int. Cl.: C07K 14/025, A61K 47/48

(54) **Fragmente des Virus Proteins 2 oder 3 des Polyomavirus als Fähren für Wirkstoffe**

(30) Priorität: 10.04.1999 DE 19916224
(62) Teilanmeldung aus: 00922458.5
(71) Anmelder: November Aktiengesellschaft Gesellschaft für Molekulare Medizin, 91056 Erlangen (DE)
(72) Erfinder: Bertling, Wolf, 91056 Erlangen (DE); Reiser, Christian, 96047 Bamberg (DE); Walter, Jürgen,Dr., 69118 Heidelberg (DE)
(74) Vertreter: Gassner, Wolfgang

(57) **Zusammenfassung**

Die Erfindung betrifft ein synthetisches biologisch aktives Molekül zum Verankern eines Wirkstoffs am Virusprotein 1 (VP1) des Polyoma-Virus, wobei eine vom C-terminalen Ende des Virus Proteins 2 (VP2) bzw. 3 (VP3) des Polyoma-Virus abgeleitete Aminosäuresequenz A1 an ihrem einen Ende mit einem Wirkstoff verbunden ist.

## Beschreibung

Die Erfindung betrifft ein synthetisches biologisch aktives Molekül und ein Verfahren zu dessen Herstellung.

Aus Chen, X.S., Stehle, T., und Harrison, S.C. (1998): Interaction of polyomavirus internal protein VP2 with the major capsid protein VP1 and implications for participation of VP2 in viral entry, The EMBO Journal, Vol. 17, No. 12, pp. 3233-3240 sind die für eine Verankerung von den Virusproteinen VP2 bzw. VP3 des Polyomavirus am Virusprotein 1 verantwortlichen Wechselwirkungen bekannt. Danach erfolgt die Verankerung im Bereich des C-terminalen Endes des VP2 bzw. VP3.

Aus der US 4,950,599 ist es bekannt, dass das Polyomavirus zum Transport von Wirkstoffen in Zellen geeignet ist. Ferner ist es aus der DE 196 18 797 A1 bekannt, dass ein vom Polyomavirus abgeleitetes Kapsomer sich zum Transport von molekularer Substanz in Zellen eignet.

Aus der EP 0 259 149 A2 ist es bekannt, das innere Kapsidprotein VP6 aus Rotavirus als immunologisches Trägermolekül und als Vakzin zur Stimulierung der Immunantwort gegen Rotavirusinfektionen zu verwenden. Dabei werden immunogene Peptide über nicht näher definierte Peptid-Peptidwechselwirkungen an VP6 gebunden. VP6 bildet hier kein strukturiertes Kapsomer, sondern zeigt im Gegenteil einen ausgeprägten strukturellen Polymorphismus. Das VP6 liegt als Monomer oder in oligomerer Form vor. Es können sich aus oligomerem VP6 zwar Partikel bilden. Dabei handelt es sich jedoch nicht um ein Kapsid oder ein Kapsomer, sondern um ein unstrukturiertes Trägerprotein.

In Redmond, M.J. et al. (1991): Rotavirus particels function as immunological carriers for the delivery of peptides from infectious agents and endogenous proteins, Mol. Immunol. 28, 269-278 ist die Verwendung des inneren Kapsidproteins VP6 aus Rotavirus als Transportpartikel bekannt. Dabei ist das VP6 über ein von der Peptidsequenz des rotaviralen Proteins VP4 abgeleitetes Bindeprotein an immunogene Peptide oder Proteine gebunden. Ein an die von VP4 abgeleitete Peptidsequenz gekoppeltes Antigen befindet sich an der Außenseite des Transportpartikels und ist daher vor Abbau nicht geschützt.

In GB 22 57 431 A ist die Verwendung eines chimären Proteins beschrieben, das vom Hüllprotein des Phagen MS-2 abgeleitet ist. Dieses Protein kann Kapside bilden. Daran gekoppelte antigene Peptide oder dgl. sind an die Außenseite des Kapsids gebunden. Bei einer spontanen Assembierung des chimären Proteins während der Expression in E.coli besteht ein hohes Kontaminationsrisiko durch bakterielle DNA oder Proteine.

Aus der DE 43 35 025 A1 ist ein endosomolytisch wirksames virusähnliches Partikel bekannt, das auf seiner äußeren Oberfläche mit membranaktiven Peptiden modifiziert ist. Die Herstellung dieses Partikels ist aufwendig.

Aufgabe der Erfindung ist es, die Nachteile nach dem Stand der Technik zu beseitigen. Es soll insbesondere eine einfache Möglichkeit zur gezielten Assoziation von Wirkstoffen mit VP1 des Polyomavirus angegeben werden.

Diese Aufgabe wird durch die Merkmale der Ansprüche 1 und 8 gelöst. Zweckmäßige Ausgestaltungen ergeben sich aus den Merkmalen der Ansprüche 2 bis 7.

In der Beschreibung finden die folgenden Definitionen Anwendung:

Abgeleitete Aminosäuresequenz: Aminosäuresequenz, die gegenüber der Aminosäuresequenz, von der sie abgeleitet ist, unverändert ist oder sich davon durch Aminosäureaustausche, Insertionen oder Deletionen unterscheidet.

C-terminales Ende: Bereich oder Region am C-Terminus.

Synthetisches Molekül: Künstlich hergestelltes Molekül.

Koppeln bzw. Ankoppeln: Kovalentes oder nicht kovalentes Binden. Ein nicht kovalentes Binden kann z.B. über eine Chelat-Bindung erfolgen.

Gentechnik: Technik, die Verfahren zum Einbringen definierter Nukleinsäuren in Zellen umfaßt.

Nach Maßgabe der Erfindung ist ein synthetisches biologisch aktives Molekül vorgesehen, wobei eine vom C-terminalen Ende des Virus Proteins 2 (VP2) bzw. 3 (VP3) des Polyomavirus abgeleitete Aminosäuresequenz (A1) mit einem Wirkstoff verbunden ist.

Das vorgeschlagene synthetische biologisch aktive Molekül ermöglicht auf einfache Weise die gezielte Assoziation von Wirkstoffen mit VP1 des Polyomavirus. Dabei bildet sich ein strukturiertes Kapsomer aus. Unter Verwendung desselben ist eine einfache Herstellung von Kapsiden als universelle Träger für Wirkstoffe möglich.

Vorteilhafterweise besteht die Aminosäuresequenz (A1) aus 10 bis 55, vorzugsweise aus 28 bis 38, Aminosäuren. Die Beschränkung auf eine relativ kurze Aminosäuresequenz vereinfacht und verbilligt die Herstellung des synthetischen biologisch aktiven Moleküls.

Die Aminosäuresequenz entspricht zweckmäßigerweise zumindest abschnittsweise der VP2-Sequenz von der Aminosäureposition 250 bis 319, vorzugsweise von der Aminosäureposition 260 bis 300, besonders vorzugsweise von der Aminosäureposition 287 bis 297. Diese Aminosäuresequenz gewährleistet eine sichere Verankerung am VP1.

Beim synthetischen biologisch aktiven Molekül weist die Aminosäuresequenz (A1) vorzugsweise Aminosäuren in folgender Sequenz auf:

Trp Met Leu Pro Leu Ile Leu Gly Leu Tyr Gly
1               5                   10

Der Wirkstoff ist vorzugsweise über einen Linker an die Aminosäuresequenz (A1) gebunden. Der Linker kann aus mindestens einer Aminosäure, einem Peptid, Protein, Lipid oder dgl. gebildet sein. Der Wirkstoff kann aus der folgenden Gruppe ausgewählt sein: Nukleinsäure, Oligonukleotid, Protein, Peptid, peptidische Substanz, PNA, Modifikationen der genannten Substanzen und niedermolekulare pharmazeutische Wirkstoffe. Geeignet sind insbesondere solche Wirkstoffe, welche über eine der unten erwähnten reaktiven Gruppen an die Aminosäuresequenz ankoppeln.

Das synthetische biologisch aktive Molekül kann gekoppelt an eine von VP1 des Polyomavirus abgeleitete Aminosäuresequenz vorliegen und/oder Bestandteil eines Medikaments sein.

Nach weiterer Maßgabe der Erfindung ist ein Verfahren zur Herstellung des erfindungsgemäßen synthetischen biologisch aktiven Moleküls mit folgenden Schritten vorgesehen:
a) Bereitstellen einer vom C-terminalen Ende des Virus Proteins 2 (VP2) bzw. 3 (VP3) des Polyomavirus abgeleiteten Aminosäuresequenz (A1), wobei die Aminosäuresequenz (A1) ein Mittel zum Ankoppeln aufweist und
b) Binden des Wirkstoffs über das Mittel zum Ankoppeln an die Aminosäuresequenz (A1).

Das Mittel zum Ankoppeln kann als Aminosäure Glycin, Cystein oder über Lysin gebundenes Glycin aufweisen. Vorteilhafterweise ist das Mittel zum Ankoppeln eine an das N- oder C-terminale Ende der Aminosäuresequenz (A1) gebundene weitere, vorzugsweise synthetisch hergestellte, Aminosäuresequenz (A2).

Das synthetische biologisch aktive Molekül kann, zumindest teilweise, gentechnisch hergestellt werden. Dabei kann die Aminosäuresequenz (A1), die weitere Aminosäuresequenz (A2) und der Wirkstoff ganz oder teilweise gentechnisch hergestellt sein. Die weitere Aminosäuresequenz (A2) weist zweckmäßigerweise Glycine und/oder Aminosäuren mit funktionellen Seitengruppen auf, wobei die funktionellen Seitengruppen aus der folgenden Gruppe ausgewählt sein können: Amino-, Sulfhydryl-, Carboxyl-, Hydroxyl-, Guanidinium-, Phenyl-, Indol-, Imidazol-Rest.

Das Mittel zum Ankoppeln kann eine über eine Aminosäure, vorzugsweise Glycin, Cystein oder über Lysin gebundenes Glycin, an das C- oder N-terminale Ende der Aminosäuresequenz (A1) gebundene reaktive Gruppe sein. Sie kann einen der folgenden Bestandteile aufweisen: Aminosäure mit Monobromacetylrest, Aminosäure mit Monochloracetylrest, Aminosäure mit 3-Nitro-2-Pyridinsulfenylrest (Npys). Die vorgeschlagenen reaktiven Gruppen sind universell einsetzbar. Sie eignen sich zum Ankoppeln einer Vielzahl von Wirkstoffen.

Als besonders vorteilhaft hat es sich erwiesen, dass der Wirkstoff über eine Thioether- oder Disulfidbrücken-Bindung an die Aminosäuresequenz (A1) oder an die weitere Aminosäuresequenz (A2) gebunden wird. Die Herstellung einer solchen Bindung läßt sich in der Praxis einfach bewerkstelligen. Selbstverständlich ist auch die Verwendung anderer reaktiver Gruppen denkbar. Z.B. kommt N-Succinimidyl Bromacetat oder N-Succinimidyl 3-(2-pyridyldithio) -propionat (SPDP) in Betracht.

Der Wirkstoff kann über einen Linker an die Aminosäuresequenz (A1) oder die weitere Aminosäuresequenz (A2) gebunden werden. Der Linker kann aus mindestens einer Aminosäure, einem Peptid, Protein, Lipid oder dgl. gebildet sein.

Nach weiterer Maßgabe der Erfindung ist ein Verfahren zur Herstellung des erfindungsgemäßen synthetischen biologisch aktiven Moleküls mit folgenden Schritten vorgesehen:
aa) Synthetisieren einer vom C-terminalen Ende des Virus Proteins 2 (VP2) bzw. 3 (VP3) des Polyomavirus abgeleiteten Aminosäuresequenz (A1) und
bb) Ankoppeln und Synthetisieren eines Wirkstoffs, nämlich eines Peptids, an die Aminosäuresequenz (A1),
wobei die Schritte lit. aa und lit. bb mittels Peptidsynthese oder mittels gentechnischer Methoden durchgeführt werden.

Beim Schritt lit. bb wird die Aminosäuresequenz (A1) um den Wirkstoff verlängert. Die Verlängerung bzw. das Ankoppeln des Wirkstoffs erfolgt durch fortgesetztes Ankoppeln von Aminosäureresten. Dieses Verfahren läßt sich besonders einfach durchführen.

Weitere beide vorgenannten Verfahren betreffende vorteilhafte Ausgestaltungen sind aus den Unteransprüchen ersichtlich.

### Beispiele:

### A. Synthese und Aufreinigung der Peptide:

Peptide werden durch simultane multiple Peptidsynthese (Schnorrenberg, G. und Gerhardt, H. (1989) Tetrahedron 45, 7759) an einem Peptidsynthesizerautomaten (Typ: PSSM-8 der Fa. SHIMADZU, Japan) unter Verwendung der 9-Fluorenylmethoxycarbonyl (Fmoc)/tert. Butyl (But)-Strategie nach Sheppard (Atherton, E. und Sheppard, R.C. (1989) "Solid phase peptide synthesis - a practical approach" IRL Press, Oxford) synthetisiert. Die Kopplungen werden mit jeweils 6 Äquivalenten Fmoc-geschützter Aminosäure/1-Hydroxybenzotriazol (HOBt)/12 Äquivalenten n-Methylmorpholin mit 2-(1H-Benzotriazol-1-yl)-1,1,3,3-tetramethyluronium-tetrafluorborat (TBTU) an einem polymeren Trägerharz (Typ: Tentagel S Trityl Harz, RAPP Polymere, Tübingen) bei einer Beladung von 2mMol/g Harz durchgeführt. Die Peptide enthalten C-terminal eine COOH-Gruppe.

Bei der Synthese finden folgende Schutzgruppen Verwendung: Cys (Trt), Arg (Pbf), Ser (But), Thr (But), Tyr (But), Asp (OBut), Glu (OBut), Asn (Trt), Gln (Trt), Lys (Boc), His(Trt), Trp (Boc) mit Trt: Trityl-, But: t-Butyl, OBut: t-Butylester, Boc: t-Butyloxycarbonyl und Pbf: 2,2,4,6,7-Pentamethyldihydrobenzofuran-5-sulfonyl.

Die Abspaltung aller Schutzgruppen wird mit Trifluoressigsäure (TFA)/Thioanisol/Thiokresol (95:2,5:2,5) in 3 h bei Raumtemperatur unter Zusatz von 3% Triisopropylsilan und anschließender Zugabe von 10% Trimethylchlorsilan für 1 h durchgeführt. Die Peptide liegen nach der Lyophilisation in Form ihrer Trifluoressigsäure-Salze vor.

Die Peptide werden mittels präparativer HPLC an einer Bischoff Polyencap 300 Trennsäule, 10*µ*m, 250x16 mm unter Verwendung eines Gradienten von 0,05 % Trifluoressigsäure in Wasser (Laufmittel A) nach 0,05 % Trifluoressigsäure in 80 % Acetonitril/Wasser (Laufmittel B) aufgereinigt.

Alternativ wurde eine Vydac Trennsäule vom Typ 218 TP 101522 (10-15 µm, 250x22 mm) mit einem Gradienten von 43-73 % Laufmittel B in 30 min bei einem Fluß von 15 ml/min verwendet.

Mittels Peptid-Synthese wird z.B. die folgende Aminosäuresequenz synthetisiert:

Trp Met Leu Pro Leu Ile Leu Gly Leu Tyr Gly
1               5                   10

An diese Sequenz wird die reaktive Gruppe über eine Aminosäure, vorzugsweise über Glycin, an das N-terminale Ende der Aminosäuresequenz gebunden. Die reaktive Gruppe kann aus Monochloracetyl-Glycin gebildet sein. Alternativ ist es auch möglich, einen Monobromacetylrest anzukoppeln.

### Kopplung von Monochloracetylglycin in der Festphasensynthese:

Gleiche Äquivalente Monochloracetylglycin und 1-Hydroxybenzotriazol (HOBt) werden in Dimethylformamid (DMF) gelöst, mit dem gleichen Äquivalent N,N'-Diisopropylcarbodiimid (DIC) gemischt und zu dem Peptidharz gegeben. Monochloracetylglycin liegt dabei im Vergleich zum beladenen Peptidharz im Überschuß vor. Die Reaktion erfolgt unter gelegentlichem Rühren und sollte mindestens 1 h andauern.

Die reaktive Gruppe ermöglicht eine kovalente Bindung des so hergestellten biologisch aktives Moleküls mit einem Wirkstoff, z.B. einem Peptid, das eine freie SH-Gruppe aufweist. Durch die Reaktion der SH-Gruppe mit dem Chloratom der Monochloracetyl-Gruppe bildet sich eine stabile Thioether Verbindung gemäß der folgenden Reaktionsgleichung:

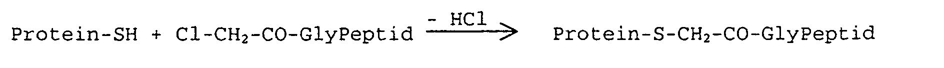

### Konjugatbildung zwischen Monochloracetyl-modifiziertem Ankerpeptid und Peptid mit endständigem Cystein:

Monochloracetyl-modifiziertes Ankerpeptid wird im Überschuß gegenüber dem zu konjugierenden Peptid eingesetzt. Die Reaktion erfolgt in 0,1 M NaHCO₃ zwischen pH 7-8 bei RT. Bei geringer Löslichkeit des Peptids oder des Ankers in wäßriger Lösung wird die Konjugatbildung in 4M Guanidinhydrochlorid, pH 8.0 durchgeführt (Lindner, W. und Robey, F.A. (1987) Int. J. Pept. Protein Res. 30, 794-800). Alternativ läßt sich der organische Lösungsmittelanteil, z.B. DMSO im Reaktionsansatz erhöhen. Zur Vermeidung unerwünschter Nebenprodukte können wasserlösliche Phosphine als Reduktionsmittel zugegegben werden.

Wahlweise läßt sich die Konjugationsreaktion auch unter folgenden Bedingungen durchführen. Das monochloracetylierte Ankerpeptid und ein Peptid mit endständiger SH-Gruppe werden in 1-Methyl-2-pyrrolidon in Gegenwart eines etwa 10-fachen Überschusses an Diisopropylethylamin und eines ca. 5-fachen Überschusses Tributylphosphin bei RT inkubiert. Nach der Reaktion wird H₂O zugefügt und das Produkt durch Zugabe von Ether präzipitiert und durch Gelfiltration aufgereinigt (Defoort, J.P., Nardelli, B., Huang, W. und Tam, J.P.(1992) Int. J. Protein Res. 40, 214-221).

Wahlweise läßt sich die Konjugationsreaktion auch folgendermaßen durchführen. Das die SH-Gruppe enthaltende Peptid wird in 0.2 M Phosphatpuffer, 10 mM EDTA, pH 7.4 gelöst. Hierzu wird das in Dimethylformamid gelöste Monochloracetylmodifizierte Ankerpeptid gegeben. Nach der Reaktion erfolgt die Aufreinigung durch Gelfiltration oder RP-HPLC (Zhang, L. und Tam, J.P. (1997) J. Am. Chem. Soc. 119, 2363-2370).

Zur Herstellung von isolierten VP1 Pentameren wird VP1 in E.coli als rekombinantes Protein mit einem N-terminalen 6 x Histidin Affinitäts-Tag (=His-Tag) exprimiert. Das Protein wird über eine Ni-NTA Affinitätschromatographie gereinigt.

Der His-Tag wird durch eine Faktor Xa-Behandlung entfernt. Das Protein wird in einem SDS-PAGE-Gel mit anschließender Coomassie-Färbung analysiert.

Dieses Ausgangsmaterial (VP1-Protein in 20mM Hepes, pH 7,3, 1 mM EDTA, 200 mM NaCl, 5 % Glycerin) wird in centricon 100 (Amincon) konzentriert und über FPLC-Gelfiltration (Superdex 200) mit einem Laufpuffer (50 mM Tris, 0,15 M NaCl, 5 mM EDTA, pH 8,5) in die hochmolekulare Kapsidfraktion und die Pentamer-Untereinheiten (Molekulargewicht: etwa 225 kD) getrennt. Beide Fraktionen werden in centricon 100 konzentriert. lodacetamid (SIGMA) wird in einem 10-fach molaren Überschuss zu der die Pentamere enthaltenden Lösung gegeben, um potentiell reaktive SH-Gruppen zu blockieren. Die Reaktion erfolgt für 2 Stunden bei Raumtemperatur. Die modifizierte Pentamerfraktion wird über Gel-Filtration von überschüssigem Iodacetamid getrennt. Mit Hilfe von VP1 spezifischen Antikörpern und einer Affinitätsmatrix (Protein A Support der Firma BIO-RAD) werden VP1-spezifische monoklonale Antikörper adsorbiert. Mit der Antikörper-beschichteten Matrix wird die gereinigte Pentamerfraktion präzipitiert. In einem weiteren Inkubationsschritt wird die Pentamer-Matrix mit der Ankersequenz versetzt. Die Proben werden in einem SDS-Polyacrylamid-Gel (12.5%) analysiert.

### Konjugatbildung zwischen Npys-modifiziertem Anker-Peptid und Peptid mit endständigem Cystein:

Wahlweise kann neben der Reaktion zwischen einem monochlor- oder monobromacetyliertem Ankerpeptid und einem Peptid mit endständigem Cystein unter Ausbildung eines Thioethers die Konjugatbildung zwischen Ankerpeptid und Peptidsequenz auch über die 3-Nitro-2-pyridinsulfenyl-(=Npys) Gruppe an einem endständigen Cystein des Ankers und einer SH-Gruppe des zu koppelnden Peptids erfolgen. Statt eines monochloracetylierten Glycin wird hierzu ein Npys-modifiziertes Cystein an die Ankersequenz N-terminal gekoppelt. Dabei handelt es sich um ein_sogenanntes aktiviertes Disulfid, welches in der Lage ist mit Thiolen, wie z.B. Cysteinen, unter Bildung eines unsymmetrischen Disulfids zu reagieren. Dabei kommt es zur Abspaltung von 3-Nitro-2-thiopyridon, dessen UV Maximum bei 329 nm eine spektroskopische Untersuchung der Kinetik der Reaktion zwischen beiden Verbindungen gestattet.

Für die Reaktion werden folgende Bedingungen gewählt (Albericio, F., Andreu, D., Giralt, E., Navalpotro, C., Pedroso, E., Ponsati, B. und Ruiz-Gayo, M. (1989) Int. J. Peptide Res. 34, 124-128). Das in 0.1 M Natriumacetat, 0.1 M Natriumchlorid, pH 4.5 gelöste zu koppelnde Peptid mit endständiger SH-Gruppe wird mit dem Npys-modifizierten Peptid versetzt, anschließend der pH auf 5.0 eingestellt und für mindestens 12 h unter Rühren inkubiert. Danach wird der pH durch Zugabe von 1N NaOH auf 7.0 eingestellt und nochmals 3 h inkubiert. Nach der Reaktion wird der Ansatz gegen 10 mM NaHCO₃ dialysiert.

Die Reaktion läuft optimal in einem pH Bereich zwischen 4.5 und 7.0 ab. Diese Bedingungen gewährleisten eine Minimierung unerwünschter Nebenreaktionen, wie z.B. die Ausbildung von symmetrischen Disulfiden zwischen den zu koppelnden Peptidmolekülen oder eine Abspaltung der Npys-Gruppe. Das Npysmodifizierte Peptid sollte bei der Reaktion im Überschuß gegenüber dem zu konjugierenden Peptid vorliegen (Albericio, F., Andreu, D., Giralt, E., Navalpotro, C., Pedroso, E., Ponsati, B. und Ruiz-Gayo, M. (1989) Int. J. Peptide Res. 34, 124-128).

Beispiele der Erfindung sind in den folgenden Sequenzprotokollen dargestellt:

Das Sequenzprotokoll 1 zeigt eine von VP2 des Polyomavirus, Position 287-297, abgeleitete Aminosäuresequenz. Sie dient im synthetischen biologisch aktiven Molekül als Anker zum Verankern des Wirkstoffs am VP1.

Das Sequenzprotokoll 2 zeigt ein erstes Ausführungsbeispiel eines synthetischen biologisch aktiven Moleküls. Die vom HIV-1 abgeleitete Peptidsequenz entspricht den Positionen 1-21; die als Anker wirkende angekoppelte Aminsosäuresequenz besetzt die Positionen 22-33. Sie ist vom VP2 des Polyomavirus abgeleitet.

Im Sequenzprotokoll 3 ist ein weiteres Beispiel für eine als Anker geeignete Aminosäuresequenz gezeigt.

Das Sequenzprotokoll 4 zeigt die Sequenz des VP2 vom Polyomavirus. Daraus sind die als Anker geeigneten Sequenzen zwischen den Positionen 250 und 300 ersichtlich.

In den Sequenzprotokollen 5 und 6 sind weitere synthetische biologisch aktive Moleküle gezeigt. Sie können mit VP1 des Polyomavirus gekoppelt und anschließend zur Behandlung einer HIV-Infektion in die infizierten Zellen eingeschleust werden.

## Patentansprüche

1. Kapsid als Träger für einen Wirkstoff, umfassend ein strukturiertes Kapsomer, wobei ein synthetisches biologisch aktives Molekül aus einem Wirkstoff und einer vom C-terminalen Ende des Virus Proteins 2 (VP2) bzw. 3 (VP3) des Polyomavirus abgeleiteten Aminosäuresequenz (A1) gebildet ist, wobei der Wirkstoff an die Aminosäuresequenz (A1) gebunden ist, wobei der Wirkstoff mittels der Aminosäuresequenz (A1) mit dem Virus Protein 1 (VP1) des Polyomavirus unter Ausbildung des strukturierten Kapsomers assoziiert ist.

2. Kapsid nach Anspruch 1, wobei die Aminosäuresequenz (A1) aus 10 bis 55, vorzugsweise aus 28 bis 38, Aminosäuren besteht.

3. Kapsid nach einem der vorhergehenden Ansprüche, wobei die Aminosäuresequenz (A1) zumindest abschnittsweise der VP2-Sequenz von der Aminosäureposition 250 bis 319, vorzugsweise von der Aminosäureposition 260 bis 300, besonders vorzugsweise von der Aminosäureposition 287 bis 297, entspricht.

4. Kapsid nach einem der vorhergehenden Ansprüche, wobei die Aminosäuresequenz (A1) Aminosäuren in folgender Sequenz aufweist:

Trp Met Leu Pro Leu Ile Leu Gly Leu Tyr Gly
1               5                   10

5. Kapsid nach einem der vorhergehenden Ansprüche, wobei der Wirkstoff über einen Linker an die Aminosäuresequenz (A1) gebunden ist.

6. Kapsid nach einem der vorhergehenden Ansprüche, wobei der Wirkstoff aus der folgenden Gruppe ausgewählt ist: Nukleinsäure, Oligonukleotid, Protein, Peptid, peptidische Substanz, PNA, Modifikationen der genannten Substanzen und niedermolekulare pharmazeutische Wirkstoffe.

7. Kapsid nach einem der vorhergehenden Ansprüche, wobei das synthetische biologisch aktive Molekül an eine von VP1 des Polyomavirus abgeleitete Aminosäuresequenz gekoppelt ist.

8. Verwendung von VP1 des Polyomavirus gekoppelt mit einem synthetischen biologisch aktiven Molekül, gebildet aus einem Wirkstoff und einer vom C-terminalen Ende des Virus Proteins 2 (VP2) bzw. 3 (VP3) des Polyomavirus abgeleiteten Aminosäuresequenz (A1), wobei an die Aminosäuresequenz (A1) der Wirkstoff gebunden ist, so dass der Wirkstoff mittels der Aminosäuresequenz (A1) mit dem Virus Protein 1 (VP1) des Polyomavirus unter Ausbildung eines strukturierten Kapsomers assoziierbar ist, zum Einschleusen des synthetischen biologisch aktiven Moleküls in Zellen, wobei jedes Verfahren zur chirurgischen oder therapeutischen Behandlung des menschlichen oder tierischen Körpers und jedes Diagnostizierverfahren, welches am menschlichen oder tierischen Körper vorgenommen wird, ausgenommen ist.


SEQUENZPROTOKOLL

<110> november Aktiengesellschaft Gesellschaft für molekulare Medizin

<120> Synthetisches biologisch aktives Molekuel

<130> 401043GA

<140>
<141>

<150> DE 199 16 224.7
<151> 1999-04-10

<160> 6

<170> PatentIn Ver. 2.1

<210> 1
<211> 11
<212> PRT
<213> Polyomavirus sp.

<220>
<222> (287) ... (297)
<223> Sequenz aus VP2

<400> 1
Trp Met Leu Pro Leu Ile Leu Gly Leu Tyr Gly
  1               5                  10

<210> 2
<211> 33
<212> PRT
<213> Künstliche Sequenz

<220>
<223> Beschreibung der künstlichen Sequenz: Aminosäuren 1-21 RT
      aus HIV-1; Aminosäuren 22-33 VP2-Sequenz aus Polyomavirus

<400> 2

Leu Ala Glu Asn Arg Glu Ile Leu Lys Glu Pro Val His Gly Val Tyr
  1               5                  10                  15

Tyr Asp Pro Ser Lys Pro Asp Trp Met Leu Pro Leu Ile Leu Gly Leu
             20                  25                  30

Tyr

<210> 3
<211> 34
<212> PRT
<213> Polyomavirus sp.

<220>
<222> (263) ... (296)
<223> Sequenz aus VP2

<400> 3
Gln Asp Glu Ser Gly Glu Val Ile Lys Phe Tyr Gln Ala Gln Val Val
  1               5                  10                  15

Ser His Gln Arg Val Thr Pro Asp Trp Met Leu Pro Leu Ile Leu Gly
             20                  25                  30

Leu Tyr

<210> 4
<211> 319
<212> PRT
<213> Polyomavirus sp.

1

<220>
<222> (1) ... (319)
<223> Vollständige Sequenz von VP2

<400> 4
Met Gly Ala Ala Leu Thr Ile Leu Val Asp Leu Ile Glu Gly Leu Ala
1               5                   10                  15

Glu Val Ser Thr Leu Thr Gly Leu Ser Ala Glu Ala Ile Leu Ser Gly
            20                  25                  30

Glu Ala Leu Ala Ala Leu Asp Gly Glu Ile Thr Ala Leu Thr Leu Glu
        35                  40                  45

Gly Val Met Ser Ser Glu Thr Ala Leu Ala Thr Met Gly Ile Ser Glu
    50                  55                  60

Glu Val Tyr Gly Phe Val Ser Thr Val Pro Val Phe Val Ser Arg Thr
65                  70                  75                  80

Ala Gly Ala Ile Trp Leu Met Gln Thr Val Gln Gly Ala Ser Thr Ile
                85                  90                  95

Ser Leu Gly Ile Gln Arg Tyr Leu His Asn Glu Glu Val Pro Thr Val
            100                 105                 110

Asn Arg Asn Met Ala Leu Ile Pro Trp Arg Asp Pro Ala Leu Leu Asp
        115                 120                 125

Ile Tyr Phe Pro Gly Val Asn Gln Phe Ala His Ala Leu Asn Val Val
    130                 135                 140

His Asp Trp Gly His Gly Leu Leu His Ser Val Gly Arg Tyr Val Trp
145                 150                 155                 160

Gln Met Val Val Gln Glu Thr Gln His Arg Leu Glu Gly Ala Val Arg
                165                 170                 175

Glu Leu Thr Val Arg Gln Thr His Thr Phe Leu Asp Gly Leu Ala Arg
            180                 185                 190

Leu Leu Glu Asn Thr Arg Trp Val Val Ser Asn Ala Pro Gln Ser Ala
        195                 200                 205

Ile Asp Ala Ile Asn Arg Gly Ala Ser Ser Ala Ser Ser Gly Tyr Ser
    210                 215                 220

Ser Leu Ser Asp Tyr Tyr Arg Gln Leu Gly Leu Asn Pro Pro Gln Arg
225                 230                 235                 240

Arg Ala Leu Phe Asn Arg Ile Glu Gly Ser Met Gly Asn Gly Gly Pro
                245                 250                 255

Thr Pro Ala Ala His Ile Gln Asp Glu Ser Gly Glu Val Ile Lys Phe
            260                 265                 270

Tyr Gln Ala Gln Val Val Ser His Gln Arg Val Thr Pro Asp Trp Met
        275                 280                 285

Leu Pro Leu Ile Leu Gly Leu Tyr Gly Asp Ile Thr Pro Thr Trp Ala
    290                 295                 300

Thr Val Ile Glu Glu Asp Gly Pro Gln Lys Lys Lys Arg Arg Leu
305                 310                 315

<210> 5
<211> 21
<212> PRT
<213> Künstliche Sequenz

<220>
<223> Beschreibung der künstlichen Sequenz: Aminosäuren 1-20 RT
      aus HIV-1 (Accession Nummer AJ006287) und ein
      zusaetzliches C-terminales Cys

<400> 5
Leu Ala Glu Asn Arg Glu Ile Leu Lys Glu Pro Val His Gly Val Tyr
1               5                   10                  15

Tyr Asp Pro Ser Cys
            20


<210> 6
<211> 17
<212> PRT
<213> Human immunodeficiency virus type 1

<220>
<223> Teilsequenz von GAG aus HIV-1 (Accession Nummer
      AJ006287)

<400> 6
Gly Ser Glu Glu Leu Arg Ser Leu Tyr Asn Thr Val Ala Thr Leu Tyr
1               5                   10                  15

Cys